# EUROPEAN PATENT APPLICATION

(11) **EP 4 571 767 A1**
(43) Date of publication of application: **18.06.2025**
(21) Application number: 23216790.8
(22) Date of filing: 14.12.2023
(51) Int. Cl.: G16H 40/20

(54) **CLINICAL DECISION SUPPORT SYSTEM AND METHOD**

(71) Applicant: Koninklijke Philips N.V., 5656 AG Eindhoven (NL)
(72) Inventor: BOUWMAN, Wilbert, Eindhoven (NL); SERLIE, Iwo, Eindhoven (NL); BULUT, Murtaza, 5656AG Eindhoven (NL); RASMIJN, Anita, Eindhoven (NL)
(74) Representative: Philips Intellectual Property & Standards

(57) **Abstract**

A system for use in clinical decision support for transforming one or more steps of a clinical workflow to modify an agent allocated to perform the step of the workflow.

## Description

### FIELD OF THE INVENTION

The present invention relates to the field of computer-based clinical decision support.

### BACKGROUND OF THE INVENTION

One function that may be performed by clinical decision support (CDS) systems is to support care providers in following pre-defined clinical workflows in the monitoring and treatment of patients. A workflow may be defined by a workflow specification which defines a plurality of steps linked by a process flow structure, e.g. defining an order of steps and possibly defining a conditional logic structure linking steps. A clinical decision support system may track progress or status within a workflow for a patient and support a clinician in automatically caching and trending relevant biological parameters at appropriate times and in prompting or triggering patient interaction events, to be performed either by a clinician or by a patient interaction device such as a sensing/measurement device or a treatment device (e.g. an IV).

Within this context, an important factor is the management and optimization of the workflows whose implementation is being supported by the CDS system.

With regards to the construction and design of clinical workflows, the underlying reasoning and logic can originate from a number of different sources, as will now be briefly discussed.

Clinical practice guidelines (CPGs) are used for clinical decision making and as a standard framework for measuring quality of care. They can be defined as "systematically developed statements to assist practitioner and patient decisions about appropriate health care for specific clinical circumstances" (Field et.al., Institute of Medicine, National Academies Press; 1990).

Hospital protocols are procedural descriptions of how CPGs are applied. They are the guidelines used by the institution to provide care and describe what is being done. They can contain workflows.

Clinical workflows are a series of interconnected events and tasks, performed by different professionals (agents), at multiple levels that can work simultaneously or sequentially to achieve a particular goal, as described in a protocol. In summary, they implement protocols in daily practice.

Having a pattern that can be relied upon, as part of health service delivery, is valuable. This minimizes the occurrence of mistakes and complications. A workflow provides an actionable implementation of processes with the aim of improving productivity and assuring outcomes.

The following five perspectives to workflow are known. The five perspectives are referred to as a workflow context in this document.

Functional. Describing what a workflow does. In workflow, tasks can be allocated to human or machine (hardware/software) agents.

Behavioral. Describing when the tasks or activities are executed. This may include a time precedence of individual tasks and triggers and the pre- and post-conditions. Human or software agents are associated with roles, roles with activities and activities with data and tools.

Informational. This relates to data elements which are consumed and produced. This may relate to the information, documents and forms that are transported between agents, as well as the files and databases that store these artifacts.

Operational. This may relate to specifying and coordinating the workflow tools and applications that support the discrete steps of the process. This aspect may describe how a workflow is implemented.

Organizational. This may relate to the organizational hierarchy, roles, security and access authorizations, the approval levels, teams and work groups, as well as a list of agents (individual people and hardware/software applications). This aspect describes which agents perform what tasks and with what tools.

Standard workflows can become strained during times of high demand in a hospital environment, particularly for example an emergency department (ED).

The negative consequences of ED crowding are well established. It is known that the ability of staff to adhere to guideline-recommended treatment at times of ED crowding decreases. This has negative consequences for patients since assessment and care may become delayed and the patient may be exposed to error. For example, there is an increased risk of blood culture contamination. By way of further example, time to critical severe sepsis therapies significantly increase and protocolized care initiation decreases. ED crowding is also associated with poor quality of care in patients with severe pain, with respect to total lack of treatment and delay until treatment. There are also negative consequences for staff in the form of stress. There may also be an increased inpatient length of stay (IPLOS) and increased emergency department length of stay (EDLOS).

There would be benefit in a structured mechanism or system to maximize adherence to CPGs, protocols, and workflows, even in difficult and unexpected conditions.

It would also be of benefit to enable flexibility in workflows to enable adaptation to changing scenarios while maintaining adherence to underlying clinical requirements. For example, it would be of benefit to enable transition to workflow variations in a dynamic setting while maintaining a systematic approach. This may improve efficiency of care administration while minimizing occurrence of mistakes and complications.

### SUMMARY OF THE INVENTION

The invention is defined by the claims.

According to examples in accordance with an aspect of the invention, there is provided a system. The system comprises a workflow store, storing a plurality of clinical workflow specifications. Each workflow specification may define a plurality of workflow steps. These may for example be linked by a process flow structure. Each workflow step may be associated in the specification with a set of workflow step components or properties. The workflow step components may include an agent indicator indicative of an agent to perform the workflow step. The workflow step components may optionally include a clinical interaction indicator indicative of a clinical interaction with the patient to be performed. Examples of clinical interactions include for instance acquiring a measurement or observation associated with the patient (i.e. obtaining information about a state of the patient), or administering or adjusting treatment to the patient or adjusting a state of the patient.

The system further includes a processing device. The processing device may include one or more processors. The processing device may be comprised by a workflow transformation subsystem for performing workflow transformation operations.

The processing device may be adapted to receive a transformation request message indicative of a request to transform an agent associated with one or more steps of a specified workflow in the workflow store from a first agent to a second agent. This may for example be received from a clinical decision support system or subsystem in some embodiments. The processing device may be further adapted to identify steps of the specified workflow for which the specified first agent is a component/property. The processing device may be adapted to, for at least one of the identified steps, send a step transformation request to a step transformer module. The step transformation request may specify the agent to be changed. The step transformer module may comprise a step transformation database which stores pre-determined transformation data packages comprising data representing a set of modifications to the same or other steps of a workflow in the event that the alternative agent performs the particular step. The processing device may be further adapted to receive from the step transformer module the transformation data package corresponding to the second agent for the specified workflow. The processing device may be further adapted to use the transformation data package to modify one or more steps of the workflow specification of the specified workflow to generate a transformed workflow specification. The processing device may be further adapted to store a data representation of the transformed workflow specification. The processing device mat be adapted to transfer a data representation of the transformed workflow specification to a decision support subsystem.

The set of modifications in the workflow package may be defined in advance, for example such that the output of the workflow step remains within acceptable bounds for the step defined by a clinical protocol of which the workflow represents a component.

In some embodiments, the set of modifications for at least one of the workflows includes changing an order of a subset of steps of the workflow.

In some embodiments, the system further comprises a workflow hosting platform for hosting one or more workflows, the workflow hosting platform comprising functionality for tracking progress through steps of each workflow being hosted.

In some embodiments, the system further comprises functionality for triggering one or more patient interaction events specified by any one or more steps of a workflow, for example a workflow being hosted by the workflow platform.

In some embodiments, the triggering of a patient interaction event may comprise: issuing a control instruction to a patient interaction device to cause the patient interaction device to perform the patient interaction; and/or generating a prompt at a user interface to prompt a user to perform the patient interaction.

In some embodiments, the system further includes functionality for receiving a patient interaction feedback signal indicative of successful completion of a patient interaction event, for example from a patient interaction device or from a user interface (in the latter case, e.g. input by a user).

In some embodiments, the system further includes a scheduler for scheduling of steps of active workflows.

In some embodiments, the set of modifications for at least one of the workflows comprises changing a measurement device to be used for acquiring a measurement in one or more of the workflow steps.

In some embodiments, the set of modifications for at least one of the workflows comprises changing a site at which one or more of the workflow steps is performed.

In some embodiments, the change of a site at which one or more of the workflow steps is performed comprises changing the workflow step from being performed in a clinical setting to being performed in a home setting. In further examples, the change in the site may comprise a change to a different clinical site or setting, such as to a different clinic within a same hospital or to a different hospital.

In some embodiments, the set of modifications may include collection of additional data, for thereby providing data redundancy.

In some embodiments, the selection of one of the possible alternative agents is performed using a transformation selector, wherein the transformation selector is adapted to select an alternative agent in dependence upon a received transformation reason code, wherein the transformation reason code is received from a user interface.

Example reasons might include: increasing capacity (to enable care of a greater number of patients), decreasing demand (decreasing number of patients that need care), or improving efficiency (making provision of care faster).

The transformation selector may be programmed with pre-defined mappings between different reasons codes and different agent transformations.

In some embodiments, the workflow transformation subsystem may be adapted to identify any of the workflow steps that can be performed at home; determine a time duration of each of the identified steps; receive from the scheduler an indication of a target time saving; and select a subset of the workflow steps whose time durations sum to a value greater than or equal to the target time saving, or a value as close as possible thereto.

In some embodiments, the scheduler is adapted to identify at least one time period which is over capacity, identify at least one workflow step scheduled for the time period, and generate a workflow transformation message indicative of a request to transform the workflow to which the identified workflow step belongs.

In some embodiments, the transformation data package indicates an additional required workflow step needed in order to change the agent for a future workflow step, and wherein the processing device is adapted to schedule the additional required workflow step at a time in advance of the future workflow step.

A further aspect of the invention provides a method for transforming clinical workflows stored in a workflow store.

The workflow store may store a plurality of clinical workflow specifications, each workflow specification defining a plurality of workflow steps. The workflow steps may for example be linked by a process flow structure in the workflow specification. Each workflow step may be associated in the specification with a set of workflow step components or properties, wherein the workflow step components include an agent indicator indicative of an agent to perform the workflow step. Optionally the workflow step components may further include a clinical interaction indicator indicative of a clinical interaction with the patient to be performed.

The method comprises:
receiving a transformation request message indicative of a request to transform an agent associated with one or more steps of a specified workflow in the workflow store from a first agent to a second agent;
identifying steps of the specified workflow for which the specified first agent is a component;
for at least one of the identified steps, sending a step transformation request to a step transformer module, the step transformation request specifying the agent to be changed, and wherein the step transformer module is communicable with a step transformation database which stores pre-determined transformation data packages comprising data representing a set of modifications to the same or other steps of a workflow in the event that the alternative agent performs the particular step;
receiving from the step transformer module the transformation data package corresponding to the second agent for the specified workflow,
using the transformation data package to modify one or more steps of the workflow specification of the specified workflow to generate a transformed workflow specification, and
storing a data representation of the transformed workflow specification.

Another aspect of the invention is a computer program product comprising machine-executable code configured, when run on a processor to cause the processor to perform a method in accordance with any example of embodiment described in this disclosure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and system in accordance with one or more embodiments of the invention;
Fig. 3 outlines an example system in accordance with one or more embodiments of the invention;
Fig. 4 outlines a further example system in accordance with one or more embodiments of the invention;
Fig. 5-outlines a further example system in accordance with one or more embodiments of the invention; and
Figs. 6-8 outline example workflow modifications for modifying a sequencing of workflow steps.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for use in managing clinical workflows. The system comprises means for implementing a workflow modification comprising modifying an agent assigned to perform a patient interaction event.

A limitation of state-of-the-art clinical decision support systems is an inability to support dynamic adaptation of workflows, for example switching between multiple alternative workflows.

Health care environments may experience variability and unpredictability of conditions, especially in an emergency department. Hospital staff typically manage pressures on health care services by making in situ adaptations and goal trade-offs to target optimum outcomes. Misalignments between demand and capacity (such as missing equipment, short staffing, and/or rapidly deteriorating condition of patients) give rise to a need for adaptation of staff activities. In the current state of the art, these adaptations are often not systematic, are error prone and often lead to suboptimal solutions.

It would be of benefit to provide a system which is able to manage a number of different workflows, provide clinician guidance regarding workflow progress and which enable real-time adaptation of workflows to accommodate changing circumstances. Ideally such modifications would be performed while maximizing adherence to CPGs, protocols, and target outcomes of workflows.

There exist state of the art decision support systems which include workflow management tools. One example is the Philips IntelliSpace portal. This tracks progress of a patient along a clinical care workflow and provides a graphical user interface displaying a graphical representation of each patient's progress along the workflow.

More particularly, Philips IntelliSpace Radiation Oncology is a protocol and workflow support system by Philips that provides workflow support. This system enables a hospital utilizing the system to configure its own workflows with customizable workflow steps. A protocol step is visualized as a protocol card which includes a representation of tasks, and data input and output requirements. A protocol step provides direct links to connected applications. The system automatically pulls data from connected systems to the protocol steps. For each workflow which forms a part of the protocol, the system provides an overview of the workflow steps and shows via a graphically displayed timeline a patient's progress along the workflow. Functionality is included to permit tasks to be automatically handed over from one user to the next.

However this existing system is not able to provide intelligent modification of workflows, for example to accommodate changes in demand or changes in available clinical staff or equipment.

In accordance with embodiments of the present invention, a system is proposed for automatically transforming a first workflow step onto a second workflow step, where a first agent (person, hardware, or software) is replaced by a second and different agent (person, hardware or software).

For example, during a busy period, it may be beneficial for a task previously assigned to a hospital staff member to be re-assigned to be performed by the patient themselves, by a tele-health person or by another staff member with different qualifications. For example, an echo imaging procedure assigned to be performed in hospital may be replaced as a workflow step by portable echo imaging which can be handled by the patient themselves to assess an identical outcome, for example to assess an empty bladder. Another example is reassigning a senior emergency-department clinician from a nonclinical role (deskwork) to a clinical role during certain high demand times.

The transformation is proposed such that the result or effect of the performed task is identical (or as close thereto as possible). In other words, the means to implement a given task are adapted but not the procedure itself.

The system can be used to provide alternative workflows to cope with emergency department crowding. However, embodiments of the invention are not limited to use in an emergency department; embodiments may find advantageous application in any clinical setting for example.

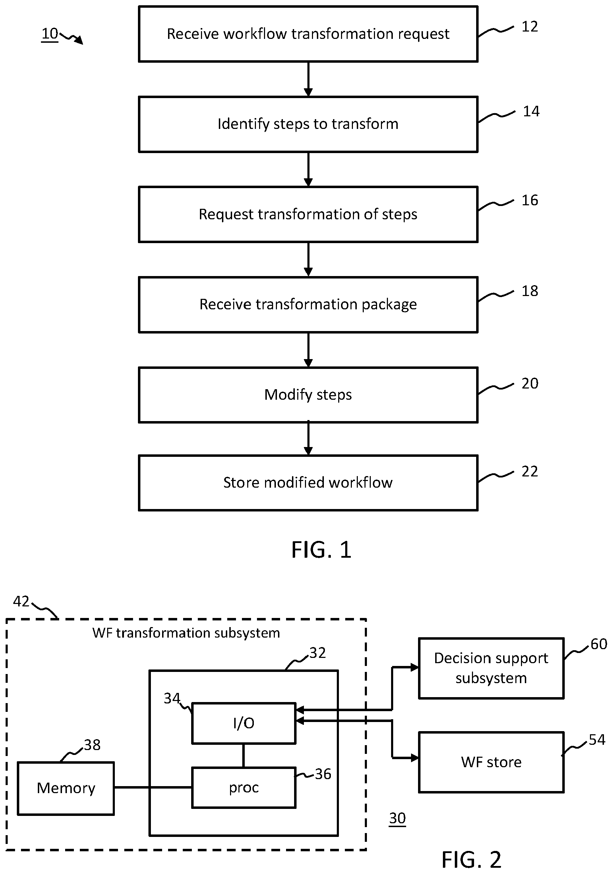

Fig. 1 outlines in block diagram form steps of an example method according to one or more embodiments. The steps will be recited in summary, before being explained further in the form of example embodiments.

The method 10 is for transforming clinical workflows stored in a workflow store. The workflow store stores a plurality of clinical workflow specifications, each workflow specification defining a plurality of workflow steps linked by a process flow structure. Each workflow step may be associated in the specification with a set of workflow step properties/components. The workflow step properties/components may include an agent indicator indicative of an agent to perform the workflow step and a clinical interaction indicator indicative of a clinical interaction with the patient to be performed.

The method comprises receiving 12 a transformation request message indicative of a request to transform an agent associated with one or more steps of a specified workflow in the workflow store from a first agent to a second agent.

The method 10 further comprises identifying 14 steps of the specified workflow for which the specified first agent is a property/component.

The method 10 further comprises, for at least one of the identified workflow steps, sending a step transformation request 16 to a step transformer module, the step transformation request defining the agent to be changed. The step transformer module may comprise a step transformation database which stores pre-determined transformation data packages comprising data representing a set of modifications to the same or other steps of a workflow in the event that the second agent performs the relevant step.

The method 10 further comprises receiving 18 from the step transformer module the transformation data package corresponding to the second agent for the specified workflow.

The method further comprises using the transformation data package to modify 20 one or more steps of the workflow specification to generate a transformed workflow specification.

The method further comprises storing 22 a data representation of the transformed workflow specification.

The method may further comprise retrieving the data representation of the transformed workflow at a decision support subsystem 60.

The method may further comprise hosting the decision support subsystem at a workflow hosting platform. The hosting the workflow may comprise tracking progress through steps of the workflow. The method may comprise generating user feedback at a user interface indicative of real-time progress through the workflow. The method may comprise triggering one or more patient interaction events specified by steps of the workflow, where the triggering a patient interaction event may comprise issuing a control instruction to a patient interaction device, and/or generating a prompt at a user interface to prompt a user to perform the patient interaction. The method may further comprise receiving a patient interaction feedback signal indicative of successful completion of a patient interaction event, for example from a patient interaction device or from a user interface (input by a human agent).

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

The processing device may be part of a workflow transformation subsystem 42 for performing transformation of workflows in accordance with one or more embodiments of the invention.

In the illustrated example of Fig. 2, the system 30 further comprises a decision support subsystem 60, details of which will be discussed later.

In the illustrated example of Fig. 2, the system 30 further comprises a workflow store 54 ("WF store") which stores a plurality of clinical workflow specifications, each workflow specification defining a plurality of workflow steps linked by a process flow structure. Each workflow step may be associated in the specification with a set of workflow step properties or components. The workflow step properties or components may include an agent indicator indicative of an agent to perform the workflow step and a clinical interaction indicator indicative of a clinical interaction with the patient to be performed. Each workflow specification stored in the datastore may correspond to a particular clinical workflow. There may be more than one workflow specification associated with each possible workflow, e.g. where there are different versions of a same workflow, e.g. different ways to achieve the same workflow outcome. In this case, each workflow specification may be indexed in the workflow datastore 54 with a workflow ID which uniquely identifies the workflow to which the workflow specification corresponds.

The system 30 may further comprise a memory 38 for storing computer program code (i.e. computer-executable code) which is configured for causing the one or more processors 36 of the processing unit 32 to perform the method 10 as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

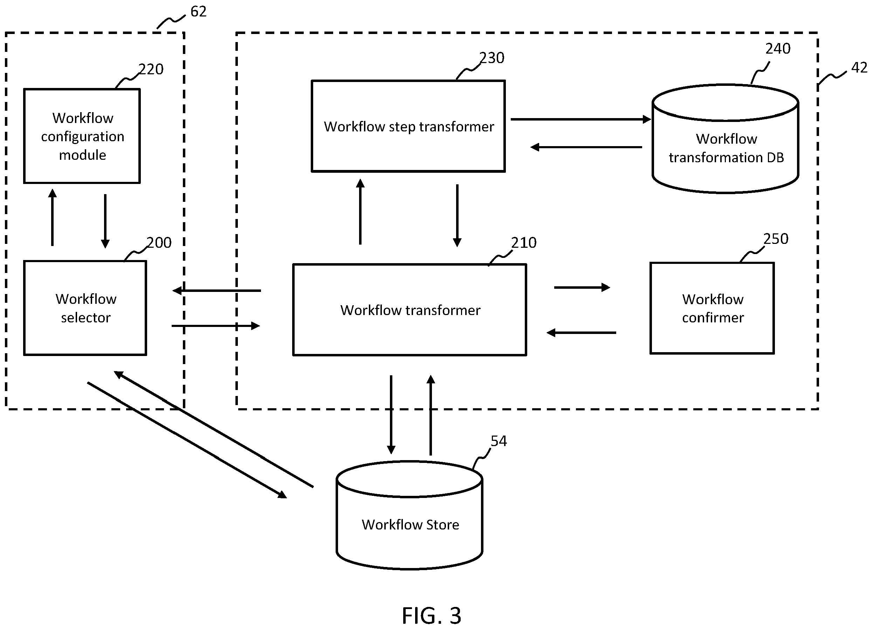

Fig. 3 outlines in more detail the process of workflow transformation in accordance with a particular set of embodiments. It will be appreciated that not all features of this particular set of embodiments are essential to the inventive concept, and are described to aid understanding and to provide an example to illustrate the inventive concepts.

Fig. 3 shows an example implementation of the workflow transformation subsystem 42 referred to previously. The workflow transformation subsystem is for transforming a first workflow to a second workflow, where the transformation includes changing an agent associated with one or more steps of the first workflow. There are different ways to achieve this, and Fig. 3 shows one example. In the example of Fig. 3, the workflow transformation subsystem includes a workflow transformer 210, a workflow step transformer 230, a workflow (step) transformation database 240 and a workflow confirmer 250.

The workflow transformer 210 receives the aforementioned request message indicative of a request to transform an agent associated with one or more steps of a specified workflow in a workflow store 54 from a first agent to a second agent. The workflow store is illustrated in Fig. 3.

The workflow transformer 210 communicates with a workflow step transformer 230 to realize transformation of one or more steps associated with the first agent (the agent to be changed). The workflow transformer may identify steps of the specified workflow for which the specified first agent is a component. For example, the workflow transformer 210 may split the workflow into a plurality of steps based on the workflow specification for the workflow and request the workflow step transformer 230 to transform a step where the agent is transformed from a first agent to a second agent, e.g. from a hospital doctor to a remote nurse.

The workflow step transformer 230 communicates with a workflow transformation (step) database 240 which stores pre-determined transformation data packages comprising data representing a set of modifications to the same or other steps of each of a plurality of workflows in the event that a specified alternative agent performs the particular step. The transformation data package may be understood as a transformation function which can be applied to a starting (first) workflow to transform it to a second (transformed) workflow. The transformation involves modifying one or more steps of the first workflow. For example, at least the workflow step specified in the request would be modified so as to change the agent. However, it will be recognized that such a change may at least in some cases necessitate a change to one or more other steps of the workflow, because steps of a workflow have a logical dependency upon one another. For example, modifying a step of a given workflow so as to be performed by a patient themselves with a measurement at home is possible if a measurement performed during an earlier step in the hospital is performed with an identical device or a device which is capable of generating a compatible/identical type of output, for reproducibility. The workflow transformation data package may thus specify changes to both the earlier and later workflow steps in this case.

The lookup of the appropriate transformation data package may be based on querying the transformation database 240. The querying may comprise querying the database based on a given formalized task associated with the workflow step being transformed. For example, any given workflow step may be associated with a target task or outcome, and wherein the workflow transformation package defines, for a specified outcome or task of a specified workflow step to be kept the same, a set of one or more modifications to the agent and/or patient interaction properties of the workflow step to achieve an equivalent outcome or task. For example, a workflow step might correspond to a task of an agent reminding a patient to visit the toilet. The workflow transformation data package may define a set of modifications to the same or other workflow steps so as to realize the same outcome or task, "agent reminds patient".

In some embodiments, the workflow (step) transformation database 240 may include more than one suitable transformation data package matching the requirements of the requested step transformation. In this case, the workflow step transformer 230 may retrieve all potential transformation data packages. The workflow step transformer may select one of the transformation data packages, based on a pre-defined selection criteria or based on user input. The criteria may be configured such that the workflow step transformer selects a best fitting transformation package.

The selected transformation data package is then sent to the workflow transformer 210. The workflow transformer applies the transformation defined by the transformation data package which may include modifying the agent and/or patient interaction properties of one or more of the steps of the original workflow specification to thereby arrive at a second, transformed workflow. For example: [remote nurse| nurse] calls patient every 3 hours.

The workflow transformer 210 thus composes a transformed workflow using the transformation package from the step transformer 230.

Optionally, to improve the validity of the transformations, in some embodiments, the workflow transformation subsystem 42 may include a workflow confirmer 250 module for confirming or validating transformed workflows generated by the workflow transformer 210. For example, an initial transformed workflow generated by the workflow transformer might be referred to as a provisional transformed workflow or candidate transformed workflow. The workflow transformer 210 may be configured to send a transformed workflow validation request to the workflow confirmer 250. The workflow confirmer 250 may be configured to check the candidate transformed workflow against one or more validation criteria. The validation criteria may be pre-defined. For example, the method may include a preliminary step of receiving at the workflow confirmer input validation criteria from a user for use in validating candidate workflows. The workflow transformer may be configured to receive a validation confirmation from the workflow confirmer 250 responsive to the candidate transformed workflow meeting the one or more validation criteria.

In some embodiments, the checking of the workflow validation criteria by the workflow confirmer may additionally comprise the following steps. This process may include the workflow transformer 210 or the workflow confirmer 250 communicating with the workflow datastore 54. The workflow datastore may store a record of workflows which have previously been validated (e.g. by the same workflow confirmer). This may include previously validated versions of the candidate transformed workflow currently under consideration. Thus, the method may include retrieving from the workflow datastore 54 previously validated versions of the candidate transformed workflow (i.e. corresponding to a same workflow, e.g. indexed according to a same workflow ID). For example, all workflows with the same steps but in a different order, or workflows with a same objective or outcome but wherein the steps are slightly modified in different versions. The set of previously validated versions of the candidate transformed workflow may be included in the validation request message in combination with the candidate transformed workflow itself. The validation assessment of the candidate transformed workflow may be performed in part based on the provided previously validated versions of the workflow.

In some embodiments, workflow datastore 54 may additionally store frequency information for each of the previously validated workflow specifications, where the frequency information is information as to frequency of past implementation of each respective validated workflow specifications. This may mean for example a number of times the respective validated workflow specification has been implemented. The method may comprise retrieving from the workflow datastore 54 stored information as to frequency of implementation of each of the previously validated versions of the candidate transformed workflow and including this information in the validation request message sent to the workflow confirmer.

Reusing workflows that were confirmed in the past supports a more structured way of working that reduces errors.

Responsive to validating the candidate transformed workflow, the workflow confirmer 250 sends the confirmed workflow to the workflow transformer 210. The workflow transformer may store the confirmed new workflow in the workflow datastore 54. Each time this transformed workflow is used, the workflow datastore 54 may be updated to maintain frequency information for the transformed workflow, indicating the number of times it has been used.

The transformed workflow, once validated, may also be communicated to the clinical decision support subsystem 60 for loading and hosting. This part of the method implementation will be described in more detail to follow.

With regards to the process of generating the transformation request message, further optional details of this process are also illustrated in Fig. 3. The generating of the workflow transformation request may be performed by the clinical decision support subsystem 60 in some embodiments, for example by a workflow selection and loading module 62 of the decision support subsystem.

In some embodiments, the workflow selection and loading module 62 includes a workflow selector module 200 and a workflow configuration module 220. The workflow selector 200 may be in communicative relationship with the workflow datastore 54, and may be operable to identify a list of available workflows stored in the datastore 54. A user interface may be controlled to provide a graphical display indicative of the available workflows, and to provide a user-control permitting user selection of one or more of the workflows to load and run. The workflow configuration module 220 may be for adjusting one or more of the workflows. The workflow configuration module may, for a user-selected one of the available workflows, be configured to identify a list of the properties/components or workflow aspects associated with each of the workflow steps. The properties for each step may include an agent for performing the workflow step, a patient interaction to be realized with the step and optionally an outcome or objective for the step. The properties might also include a location for implementation of the step. For example, a simple exemplary workflow might be a workflow for prevention of urinary retention. The steps of the workflow might comprise
a. a staff member reminds a patient every 3 hours to urinate.
b. a patient is encouraged by a staff member to take time to empty their bladder.
c. a staff member tests if a patient is retaining urine and reports the result.

The workflow configuration module may be configured to display the properties for each step of a selected workflow at the user interface. To accomplish this, the workflow datastore 54 may store for each a workflow step of each workflow specification, the properties and alternatives.

For example, for each of the steps (a)-(c) above, the agent is a hospital staff member and an alternative agent might be a remote nurse.

The workflow configuration module 220 may be configured to receive from the user interface a user-input indication of one of the workflow steps and one of the properties to modify, e.g. the agent. The user input may indicate a desired modification to the property, e.g. the agent. For example, the user may indicate a remote nurse to replace the hospital staff member as the agent.

Once the user has selected a workflow step to be modified, the workflow selector sends to the workflow transformer 210 a workflow transformation request which includes the following information: a workflow ID of the selected workflow, and an indication of agent change which is to be made.

Optionally, in some embodiments, instead of a user indicating the desired workflow property/component to modify (e.g. modification of a particular agent), the workflow selection and loading module may include functionality for determining a property, e.g. an agent, to change. For example, this might be based on system knowledge regarding time durations associated with the performance of particular workflow steps of a workflow by different possible agents.

The information of an estimated time duration required by each different possible agent to perform a workflow step may be recorded in a database, for example in the workflow transformation database 240 or a different database. For example, if a given step is done by a bed side, it might consume 30 minutes of on-site time (the time of a nurse on-site). If the same step is performed by a remote nurse, it might consume 30 min of remote nurse time, and 10 min of on-site time. If the action is performed by the patient, it might consume 40 minutes of patient time, 10 minutes of remote-nurse time, and 5 minutes of on-site nurse time. Based on these estimations, and a target objective (e.g. minimize on-site time) the system may determine an optimal alternative agent or agents to perform the step and this information may be included in the workflow transformation request.

Optionally, an additional step may be included wherein the purpose of the transformation is input by a user. This purpose information may be included in the transformation request message to the workflow transformer 210 and in the workflow step transformation request to the workflow step transformer 230. Based on this purpose criteria, agents might be searched and automatically selected (as an alternative to a user defining the agent to be changed fully manually). Example purposes may be: increasing capacity (ability to take care of more patients), decreasing demand (decreasing number of patients that need care), or improving efficiency (making the care faster).

In addition, relating to the transformation purpose, one of a set of priority levels may be selected by a user, where the priority levels indicate the importance/urgency and consequence of the requested transformation. For example:
Level 1: obtain additional staff from non-emergency department (ED) settings. This will increase capacity, but the recruited staff will not have specialist expertise in emergency care. These staff can perform regular tasks with guidance from trained ED staff.
Level 2. Obtain additional staff with ED experience (e.g., from non-clinical setting) to increase capacity and efficiency.
Level 3. During a crisis situation: Level 2 measures plus introducing fixed/forced times for board rounds. Fixed board rounds will improve communication between ED staff and coordinators. From research, this is known to improve efficiency by ensuring overall management of the department remains in accordance with current patient needs.

In some embodiments, the workflow (step) transformation database 240 may additionally store agent instructional information associated with each possible transformation for use in educating or informing the new agent with regards to performance of the relevant workflow step. For example, if the workflow step is to be performed by the patient as the new agent, the instructional information may include a manual, an instructional video and/or an understanding check list for the patient. This information may be communicated to the decision support subsystem to permit the information to be communicated to the new agent who is to perform the relevant workflow task.

To aid in understanding of the invention, there will now be presented a description of an example system architecture/environment for use in clinical decision support, within which the proposed workflow transformation method may advantageously operate. The described system, or any subset of components thereof, may be provided as a separate aspect of the invention, or as component features of any other embodiment of the invention described in this documents or defined in any claim.

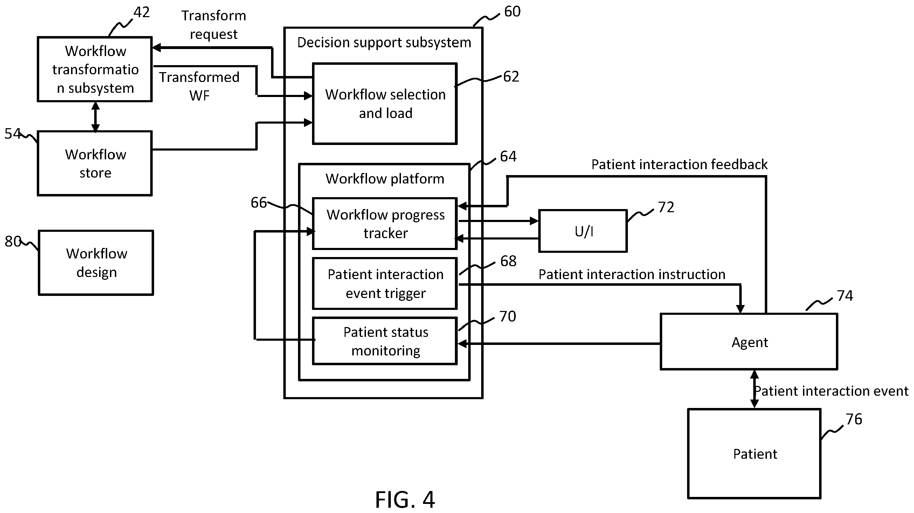

An example system is shown in Fig. 4. A system comprising any selected combination of one or more of the components of the system of Fig. 4 may be provided as an aspect of the invention. The illustrated system includes a decision support subsystem 60. The decision support subsystem 60 is for providing clinical decision support to a user, for example real-time clinical decision support.

At least one functionality provided by the clinical decision support subsystem 60 may be the hosting of clinical workflows. The system of Fig. 4 includes a workflow hosting platform 64. Hosting of a clinical workflow may comprise tracking progress through steps of the workflow specification associated with the workflow and/or managing execution of steps of the workflow specification by generating patient interaction instructions for causing or prompting execution of a patient interaction event by an agent, where the agent may be a human agent or a machine (hardware/software) agent.

The workflow hosting platform 64 includes a workflow progress tracker 66 for tracking progress through steps of each live workflow. The workflow progress tracker may be operatively coupled with a user interface 72 ("U/I") and configured to output a workflow progress status indictor to the user interface indicative of progress through the steps of the clinical workflow. One or more of the steps of a clinical workflow being hosted may indicate a need for a patient interaction, for example acquiring information about a patient state, or modifying a patient state, e.g. changing or configuring an element of treatment. The workflow hosting platform 64 may further comprise a patient interaction event trigger module 68 for generating a patient interaction instruction for causing or prompting execution of a patient interaction event by an agent 74, where the agent may be a human agent or a machine (hardware/software) agent. Fig. 4 shows the communication of the patient interaction instruction schematically. If the agent is a human, the instruction generated by the patient interaction event trigger may take the form of a user interface prompt communicated to the user interface 72 for presentation to a human agent, instructing or guiding the user to perform a specified patient interaction event. If the agent is a machine, e.g. a patient sensing/measurement device or patient therapeutic device, the instruction may comprise a machine instruction for causing the device to perform a patient interaction event such as acquiring measurement data or administering or adjusting a therapy to the patient.

The workflow progress tracker 66 may be arranged to receive patient interaction feedback generated by the agent 74 following execution of a patient interaction event, for example confirmation that a patient interaction event has been successfully completed. This can be used in tracking progression through the steps and/or logic structure of the workflow specification of a workflow being hosted. If the agent is a human agent, the patient interaction feedback may be received at the workflow progress tracker from the user interface 72 as user input information provided to the user interface by the human agent. If the agent is a machine agent, the patient interaction feedback information may be received by the workflow progress tracker 66 from the machine agent itself 74 which may generate a feedback signal responsive to successful completion of the patient interaction event.

In the example of Fig. 4, the workflow hosting platform 64 further comprises a patient status monitoring module 70 for monitoring a patient status. Thus may comprise monitoring one or more sensor measurements acquired for the patient. It may comprise receiving update information via a user interface 72 by a human agent relating to patient status. The receipt of the patient status information at the patient status monitoring module 70 is shown schematically in Fig. 4 as being received from the agent 74. If the agent is a human agent, the information may be received from the user interface 72 as user input information by the human agent. If the agent is a machine agent, the information may be received directly from the machine agent. Examples of human agent originating information might be a visual observation made by a human clinician or another physical examination result.

The patient status monitoring module 70 may communicate with the workflow progress tracker 66 and the workflow progress tracker may update a tracked progress through the steps of a hosted workflow based on information received from the patient status monitoring module. For example, reaching a threshold level of a certain physiological parameter may be a trigger condition for progressing to a next step or a different branch of the workflow specification in some examples.

Thus, in general terms, the workflow hosting platform 64 may perform live or real-time hosting of one or a plurality of workflows. Hosting of a workflow may comprise tracking progress through a set of steps of the workflow linked by a logical progression structure. For a current step of a workflow being hosted, the workflow platform may generate an output, e.g. an instruction, for triggering or prompting execution of one or more patient interaction events comprising obtaining information about a current patient status or administering or adjusting a therapeutic action associated with a patient. Feedback may be received indicative of successful completion of a patient interaction event following issuance of the patient interaction event instruction. The progress through the workflow may be updated based thereon. Patient status may also be monitored, e.g. values of one or more physiological parameters, or clinician observations, and this information may also be used to update a progress status relative to the workflow specification. Feedback regarding progress status relative to the workflow specification may be output regularly, e.g. continuously, to a user interface 72.

With regards to the workflows, the decision support system 60 may further include a workflow selection and loading module 62. The workflow selection and loading module may be communicatively coupled with the workflow store 54 which stores a plurality of different workflows, each associated with a respective workflow specification. One or more workflows may be selected and loaded from the workflow store and passed to the workflow hosting platform for hosting.

Fig. 4 illustrates the workflow transformation subsystem 42 previously discussed. The workflow transformation subsystem 42 may receive a workflow transformation request message from the decision support subsystem 60, e.g. the workflow selection and loading module 62. The workflow transformation subsystem may output a transformed workflow to the decision support subsystem 60 following execution of a workflow transformation method in accordance with any of the examples of embodiments described in this document.

Fig. 4 also schematically illustrates an optional element of the system implementation comprising design 80 and input of the workflows which populate the workflow store 54. For example, a human user may design and generate each of one or more workflow specifications. Each workflow specification may comprise a sequence of steps linked by a logic progression structure. The logic progression structure may for example include a branching logic structure, for example where progression through steps of the workflow specification depend upon one or more criteria. For example, the one or more criteria may include receiving feedback indicative of successful completion of a current or previous workflow step, and/or based on values of one or more patient clinical parameters. Embodiments of the invention may thus include a process of a user inputting one or more workflow specifications to the workflow store. Embodiments may include a process of a user designing one or more workflow specifications. The user may design the workflow specifications for example in accordance with a known clinical protocol and/or a known clinical practice guidelines.

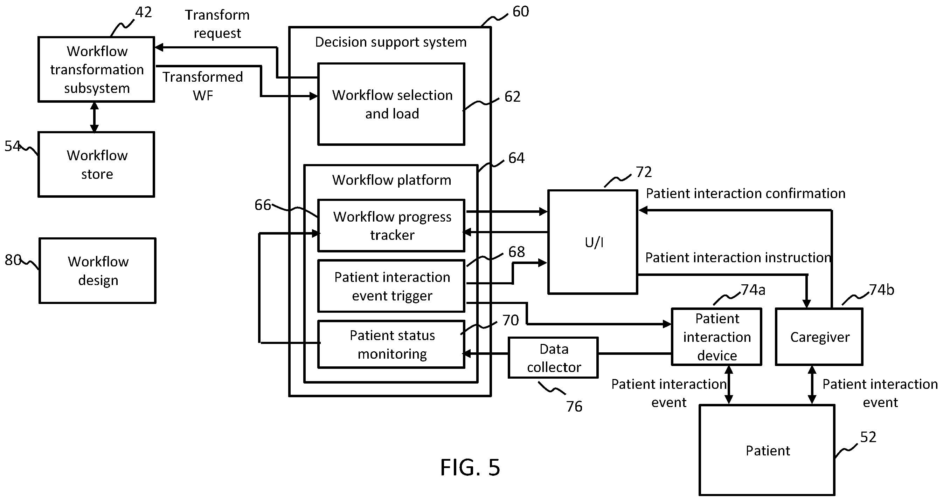

Fig. 5 shows a more detailed outline of an example system in accordance with one or more embodiments. This system may comprise the same features and functionality as described in relation to the system of Fig. 4, with the following described additions or modifications.

In the example of Fig. 5, the agent 74 role is represented with two particular examples of possible agents, one a machine agent 74a in the form of a patient interaction device, and one a human agent 74b in the form of a human caregiver, such as a hospital staff member, a remote staff member, or a family member at a home environment of the patient. Also shown is an optional data collector 76 for collecting data generated by the patient interaction device, such as measurement data. For example, this may be a hub device which collects data from a plurality of different patient interaction devices and transfers it to the patient status monitoring module 70. For example, the data collector may convert received data to a uniform format suitable for receipt by the patient status monitoring device.

From the example of Fig. 5, it can be seen how the patient interaction instruction issued by the patient interaction event trigger module 68 may take different forms depending upon whether the agent for performing the workflow step is a human agent or a machine agent. If the agent for performing the workflow step is a human agent, the instruction may be a control instruction communicated to the user interface for causing the user interface 72 to generate a user-perceptible output for prompting or instructing a human user, e.g. a human caregiver 74b, to perform the relevant patient interaction event in relation to the patient 52. If the agent to perform the workflow step is a machine agent, the instruction may be a machine-readable instruction, for example a control instruction for causing the machine agent, e.g. a patient interaction device 74a to perform the relevant patient interaction event.

In some embodiments, the set of modifications included in the workflow transformation package may include, for at least one of the workflows, changing an order of a subset of steps of the workflow. This may occur for example where the change in agent enables a step to be performed at a different time. For example, if a change in agent is from a hospital worker to a remote nurse for a given workflow step, that step could be performed at a home environment, meaning that it could be performed after discharge instead of before discharge. Thus, in this case, a hospital discharge workflow step may be accomplished before other workflow steps that are performed at home.

For example, in some embodiments, the set of modifications included in the workflow transformation package may include changing a site at which one or more of the workflow steps is performed.

In some embodiments, the change of a site at which one or more of the workflow steps is performed may comprise changing the workflow step from being performed in a clinical setting to being performed in a home setting.

In some embodiments, the system may further include a scheduler for scheduling of steps of active workflows.

In some embodiments, the method may comprise identifying any of the workflow steps that can be performed at home, determining a time duration of each of the identified steps, receiving from the scheduler an indication of a target time saving; and selecting a subset of the workflow steps whose time durations sum to a value greater than or equal to the target time saving, or a value as close as possible thereto.

In some embodiments, the scheduler may be adapted to identify at least one time period which is over capacity, identify at least one workflow step scheduled for the time period, and generate a workflow transformation message indicative of a request to transform the workflow to which the workflow step belongs.

In some embodiments, in this case, the transformation data package obtained from the transformation database may include an indication of an additional required workflow step needed in order to change the agent for a future workflow step, and wherein the method further comprises scheduling the additional required workflow step at a time in advance of the future workflow step.

Some examples will now be discussed.

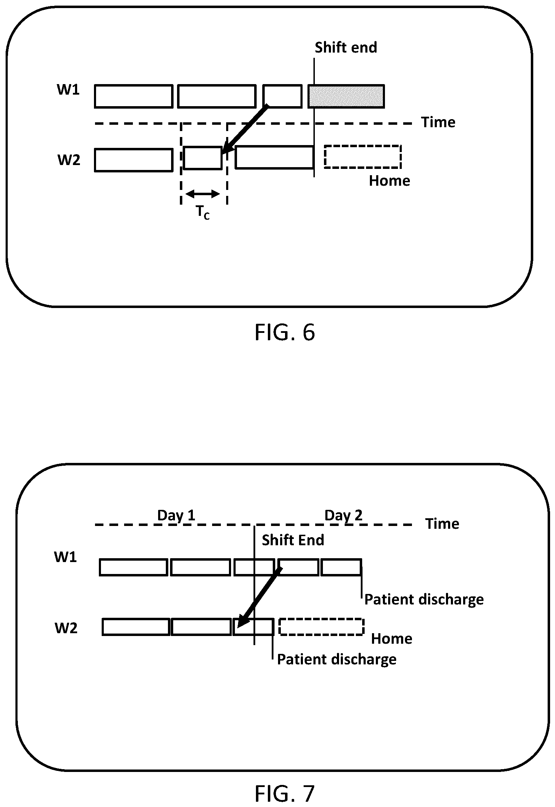

Fig. 6 shows an example in which a workflow step is shifted in time towards a new (critical) time frame, T_{c}. Due to this change, some workflow tasks could be transformed to a portable (home) setting, resulting in time saving without extending the shift of the staff.

With regards to estimation of a time duration of each workflow step, the following considerations may be taken into account. Each workflow step may have a defined purpose, where the purposes may be defined at least in part in terms of required inputs (what equipment may be needed, a required starting state of the patient, and/or actions to be performed) and required outputs (a target outcome of the procedure, e.g. measurement data, observations, or action completion such as medication). Depending upon current conditions (e.g. available inputs and inputs yet to be prepared) it can be estimated the type of additional preparations that are required. An approximate time duration for these can be estimated. Given a set of inputs, the duration to complete the whole given workflow step can also be estimated. Here, depending upon the agent, different rules may be applied (e.g. using a look up table) to estimate the duration.

In terms of technical implementation of the scheduling, one approach is to calculate a delta between the current conditions and the initialization/start conditions required to perform the step. A time required to perform the delta may be estimated. Typically, this may be based on a lookup table or other database, where the lookup table or database may be prepared in advance based on historical data. The total time required for a given workflow step may be calculated by summing the estimated time for the individual actions that are needed for that particular step.

Fig. 7 shows a second example. Here, changes in tasks which allow for earlier discharge of the patient are shown. Critical hospital workflow steps which were planned for day 2, including discharge, are moved to day one by adapting the workflow such that two of the steps of the workflow are modified to be performed at home by a home-based caregiver.

This workflow adaptation does not result in time saving at day one: it in fact extends the staff shift by 30 minutes. However, the adaptation results in substantial time saving at day 2 of 3 hours. This time saving of day 2 was due to staff savings and adaptations of tasks to a portable setting.

Fig. 6 and Fig. 7 together show different time saving strategies that might be employed. In one case (Fig. 6), time is saved to prevent extension of a current day shift. In a second case (Fig. 7), time is saved from the following day shift. The purpose of the workflow transformation may be to balance the demand/capacity ratio. Consideration of the current demand/capacity ratio, and consideration of the future ratio can be used to determine the optimal strategy. For example, if it is expected that the demand will remain high for a period that extends into the next day, it may be preferable to increase the shift duration for the current day to ease the pressure next day. If the demand is expected to reduce next day, it may be preferred to reduce the demand in the current day and move some workflow steps to the following day.

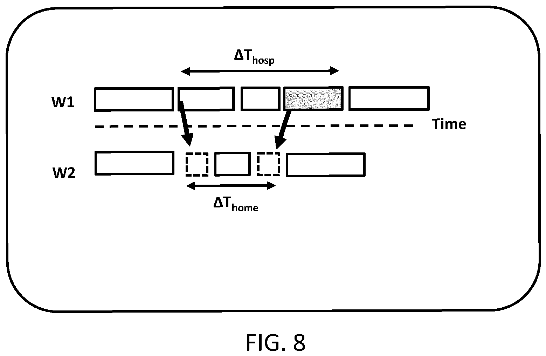

Fig. 8 shows another example in which two measurements were planned between a specified time frame, ΔTₕₒₛₚ, at a hospital environment. However, due to the high burden of the hospital, these two measurements could not be performed within the guideline (maximum time) requirements. Workflow transformation to a portable setting enables the two measurements within the guideline time requirements. The steps in the home environment were performed within a time period ΔTₕₒₘₑ which is less than ΔTₕₒₛₚ. Furthermore, it enables the minimal time requirements and earlier scheduling of the next hospital task.

In accordance with one or more embodiments, it is proposed to provide a graphical user interface configured to provide preview information indicative of available workflow transformations, which may for example indicate estimated effects on a current workflow of one or more patients. For example, the graphical user interface may be controlled to display a current workflow for one or more patients and also show one or more possible modified workflows for the same one or more patients. The one or more proposed modified workflows may be determined based on use of the workflow transformation subsystem to obtain an indication of one or more possible workflow transformations and wherein the user interface is controlled to present an output representative of these possible workflow transformations. In some examples, a difference between the current workflow and each possible modified workflow may be presented, e.g. indicating a change in the agent and indicating a time saving achieved by the modified workflow or a capacity increase achieved by the modified workflow.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system comprising:
a workflow store (54) storing a plurality of clinical workflow specifications, each workflow specification defining a plurality of workflow steps, wherein each workflow step is associated in the specification with a set of workflow step components, wherein the workflow step components include an agent indicator, indicative of an agent to perform the workflow step;
a workflow transformation subsystem (42) adapted to:
receive (12) a transformation request message indicative of a request to transform an agent associated with one or more steps of a specified workflow in the workflow store from a first agent to a second agent,
identify (14) steps of the specified workflow for which the specified first agent is a component;
for at least one of the identified steps, send (16) a step transformation request to a step transformer module, the step transformation request specifying the agent to be changed, and wherein the step transformer module is communicable with a step transformation database which stores pre-determined transformation data packages comprising data representing a set of modifications to the same or other steps of a workflow in the event that the alternative agent performs the particular step; and
receive (18) from the step transformer module the transformation data package corresponding to the second agent for the specified workflow,
use the transformation data package to modify (20) one or more steps of the workflow specification of the specified workflow to generate a transformed workflow specification, and
store (22) a data representation of the transformed workflow specification.

2. The system of claim 1, wherein the set of modifications for at least one of the workflows includes changing an order of a subset of steps of the workflow.

3. The system of claim 1 or 2, further comprising a workflow hosting platform for hosting one or more workflows, the workflow hosting platform comprising functionality for tracking progress through steps of each workflow being hosted.

4. The system of any of claims 1-3, further comprising functionality for triggering one or more patient interaction events specified by steps of a workflow.

5. The system of claim 4, wherein the triggering a patient interaction event comprises:
issuing a control instruction to a patient interaction device to cause the patient interaction device to perform the patient interaction event; and/or
generating a prompt at a user interface to prompt a user to perform the patient interaction event.

6. The system of claim 4 or 5, wherein the system further includes functionality for receiving a patient interaction feedback signal indicative of successful completion of a patient interaction event, for example from a patient interaction device or from a user interface.

7. The system of any preceding claim, wherein the system further includes a scheduler for scheduling of steps of active workflows.

8. The system of any of claims 1-7, wherein the set of modifications for at least one of the workflows comprises changing a measurement device to be used for acquiring a measurement in one or more of the workflow steps.

9. The system of any of claims 1-8, wherein the set of modifications for at least one of the workflows comprises changing a site at which one or more of the workflow steps is performed.

10. The system of claim 9, wherein the change of a site at which one or more of the workflow steps is performed comprises changing the workflow step from being performed in a clinical setting to being performed in a home setting.

11. The system of any preceding claim, wherein the workflow transformation subsystem is adapted to identify any of the workflow steps that can be performed at home,
determine a time duration of each of the identified steps;
receive from the scheduler an indication of a target time saving;
select a subset of the workflow steps whose time durations sum to a value greater than or equal to the target time saving, or a value as close as possible thereto.

12. The system of any preceding claim, wherein the scheduler is adapted to identify at least one time period which is over capacity, identify at least one workflow step scheduled for the time period, and generate a workflow transformation message indicative of a request to transform the workflow to which the workflow step belongs.

13. The system of claim 12, wherein the transformation data package indicates an additional required workflow step needed in order to change the agent for a future workflow step, and wherein the processing device is adapted to schedule the additional required workflow step at a time in advance of the future workflow step.

14. A computer-implemented method for transforming clinical workflows stored in a workflow store,
wherein the workflow store stores a plurality of clinical workflow specifications, each workflow specification defining a plurality of workflow steps, wherein each workflow step is associated in the specification with a set of workflow step components, wherein the workflow step components include an agent indicator indicative of an agent to perform the workflow step;
wherein the method comprises:
receiving a transformation request message indicative of a request to transform an agent associated with one or more steps of a specified workflow in the workflow store from a first agent to a second agent;
identifying steps of the specified workflow for which the specified first agent is a component;
for at least one of the identified steps, sending a step transformation request to a step transformer module, the step transformation request specifying the agent to be changed, and wherein the step transformer module is communicable with a step transformation database which stores pre-determined transformation data packages comprising data representing a set of modifications to the same or other steps of a workflow in the event that the alternative agent performs the particular step;
receiving from the step transformer module the transformation data package corresponding to the second agent for the specified workflow,
using the transformation data package to modify one or more steps of the workflow specification of the specified workflow to generate a transformed workflow specification, and
storing a data representation of the transformed workflow specification.

15. A computer program product comprising machine-executable code configured, when run on a processor to cause the processor to perform a method in accordance with claim 14.
